# EUROPEAN PATENT SPECIFICATION

(11) **EP 1 050 279 B1**
(45) Date of publication and mention of the grant of the patent: **21.06.2006**
(21) Application number: 00303370.1
(22) Date of filing: 20.04.2000
(51) Int. Cl.: A61B 18/14

(54) **Electrosurgical handpiece for treating tissue**
Elektrochirurgisches Handstück zur Behandlung von Gewebe
Piéce à main électrochirurgicale pour le traitement d'un tissue

(30) Priority: 03.05.1999 US 303839; 10.09.1999 US 393286; 25.10.1999 US 425313; 18.01.2000 US 483994
(43) Date of publication of application: 08.11.2000
(60) Divisional application: 05077310.0
(73) Proprietor: Garito, Jon C., Hewlett, NY 11557 (US); Ellman, Alan G., Hewlett, NY 11557 (US)
(72) Inventor: Garito, Jon C., Hewlett, NY 11557 (US); Ellman, Alan G., Hewlett, NY 11557 (US)
(74) Representative: Sturt, Clifford Mark

(56) References cited:
- WO-A-96/22742
- WO-A-97/15238
- DE-A- 4 423 216
- US-A- 5 441 499

## Description

This invention relates to an electrosurgical handpiece and an activator for an electrosurgical handpiece.

### BACKGROUND OF THE INVENTION

Electrosurgery is a common procedure for dentists, doctors, and veterinarians. Electrosurgical handpieces are commercially available that will accommodate a wide variety of electrode shapes and sizes, such as needles, blades, scalpels, balls and wire loops. Also, multi-function electrodes are available. Electrosurgery has been used for many different kinds of surgical procedures. One surgical procedure involves minimally invasive surgery (MIS), also referred to as laparoscopy, in which a small diameter cannula is inserted via an incision in the patient's body, and a fiber optics viewer (TV camera and monitor) as well as an elongated nozzle from an electrosurgical handpiece are extended through the cannula to the region of the patient where surgery is necessary, and the surgery carried out by the surgeon using the electrosurgical electrode while viewing the procedure through the viewer. Our issued US patent No. 5,304,763 describes one form of MIS.

MIS with electrosurgery has also been used for the reduction of herniated disks, by introducing a unipolar electrode via the cannula into the herniated disk region and activating the electrode for the purpose of shrinking the disk. One such system also provides for bending the end of the unipolar electrode in order to position the active end in the desired disk region. In such a procedure, care must be exercised to avoid nerve damage. In the known system, a heat sensor is built into the active unipolar electrode end for the purpose of sensing the heat generated by the electrosurgical currents and shutting off the electrosurgical currents when the heat reaches too high a level.

US 5,441,499 to Fritzsch discloses a bipolar radio-frequency surgical instrument having a tip electrode supplied with a radio-frequency voltage and a neutral electrode. The tip electrode is connected to a power source by an insulated lead disposed within a rigid tube shaft and an outer flexible tube. The tip electrode is pivotable from a position axially aligned with the tube shaft to a position which forms an angle with the tube.shaft. The neutral electrode disclosed in Fritzsch surrounds the tip electrode and has a much larger surface area than the tip electrode.

### SUMMARY OF THE INVENTION

An object of the invention is an electrosurgical handpiece that is capable of treating tissue when energised.

Another object of the invention is an electrosurgical handpiece that can be used in MIS and reduces the danger of excessive heat causing possible patient harm.

According to one aspect of the invention, there is provided an electrosurgical bipolar handpiece comprising:
(a) an elongated tubular first member having a first end and a remote flexible second end,
(b) an elongated tubular second member located within the first member, said second member comprising an electrically-insulating tube having a first end and a remote flexible second end adjacent the first member's second end,
(c) first means connected to the first member at its first end for applying electrosurgical bipolar currents to first and second wires,
(d) second means connected to the first member at its first end for selectively flexing the remote flexible end of the first member relative to the first end of the first member together with the remote flexible end of the second member,
(e) active electrically-insulated electrosurgical electrodes connected to the first and second wires and extendable at the remote flexible end, said electrosurgical electrodes flexing together with the remote flexible ends when the second means is operated while maintaining them electrically-insulated,
   characterised by
(f) electrically-insulating dividing means dividing the second member into first and second side-by- side compartments,
(g) first and second electrically-conductive wires each positioned, respectively, in the first and second compartments and electrically-insulated from each other,

The bipolar operation confines the electrosurgical currents to a small active region between the active ends of the bipolar electrode and thus reduces the possibility that excessive heat will be developed that can damage patient tissue. Moreover, the position of the active region can be controlled to avoid patent tissue that may be more sensitive to excessive heat.

This construction also allows the surgeon to position the active electrode end at the optimum location with a herniated disk to remove undesired regions and to provide controlled heat to shrink the tissue during surgery.

The construction of the invention will provide important benefits not only for MIS of herniated disks but also for other MIS procedures where controlled electrode position and controlled heat generation is of importance. Such procedures include drying, shrinking, or denaturazing tissue generally and collagen tissue in particular for such purposes as tightening or reducing the tissue.

The various features of novelty which characterize the invention are pointed out with particularity in the claims annexed to and forming a part of this disclosure. For a better understanding of the invention, its operating advantages and specific objects attained by its use, reference should be had to the accompanying drawings and descriptive matter in which there are illustrated and described the preferred embodiments of the invention.

### BRIEF DESCRIPTION OF THE DRAWINGS

In the drawings:
Fig. 1 is a perspective view of one form of an electrosurgical handpiece according to the invention fitted with a bipolar activator according to the invention and with the handle in its open position;
Fig. 2 is a side view of a variant of the handpiece of Fig. with the handle in its closed position;
Fig. 3 is a cross sectional view of the handpiece of Fig. 2 along the line 3-3;
Fig. 4 partial perspective view of the working end of the electrosurgical handpiece of Fig. 2 illustrating an early step in its manufacture;
Figs. 5 and 6 views are similar to that of Fig. 4 illustrating later steps in its manufacture,
Fig. 7 shows the assembled end of the handpiece of Fig. 2;
Figs. 8 and 9 are views similar to that of Fig. 7 fitted with two kinds of unipolar activators according to the invention;
Figs. 10 is a view similar to that of Fig. 7 fitted with a different kind of bipolar activator;
Fig. 11 is a perspective view of the device of Fig.' 7 shown connected to a bipolar adaptor and to an electrosurgical unit;
Fig. 12 illustrates use of the device of Fig. 11 in a laparoscopy procedure, namely, to reduce a herniated disk;
Fig. 13 is a perspective view of still another variant of a handpiece according to the invention comprising both a bipolar and a unipolar electrode;
Fig. 14 is an enlarged view of the working end of the handpiece of Fig. 13 with both electrodes in the retracted position;
Fig. 15 is a cross-sectional view along the line 15-15 of Fig. 13;
Fig. 16 is a perspective view of the handpiece of Fig. 13 with the bipolar electrode in its extended position and the unipolar electrode in its retracted position;
Fig. 17 is an enlarged view of the working end of the handpiece of Fig. 16;
Fig. 18 is a perspective view of the handpiece of Fig. 13 with the unipolar electrode in its extended position and the bipolar electrode in its retracted position;
Fig. 19 is an enlarged view of the working end of the handpiece of Fig. 18;
Fig. 20 is an enlarged view of the working end of a handpiece of the type shown in Fig. 13 illustrating the use of a scissors embodiment;
Fig. 21 is a perspective view of the working end of another form of a bipolar activator according to the invention. The working end is shown in its non-flexed position;
Fig. 22 is a view similar to that of Fig. 21 with the working end shown in one of its possible flexed positions;
Fig. 23 is a perspective view of the working end of another form of a bipolar activator according to the invention. The working end is shown in its non-flexed position;
Fig. 24 is a view similar to that of Fig. 23 with the working end shown in one of its possible flexed positions;
Fig. 25 is plan view of another form of handpiece according to the invention. The working end is shown in its flexed-up position;
Fig. 26 is a side view of the end of just the outer handpiece housing of another embodiment of an electrosurgical handpiece according to the invention;
Fig. 27 is a view similar to that of Fig. 26 of another form of an electrosurgical handpiece according to the invention;
Fig. 28 is a view similar to that of Fig. 29 of still another form of an electrosurgical handpiece according to the invention;
Fig. 29 is a view similar to that of Fig. 28 of yet another form of an electrosurgical handpiece according to the invention;
Fig. 30 is an enlarged cross-sectional view of loop electrode ends according to a further variant of the invention;
Fig. 31 is a cross-sectional view of the electrode end of Fig. 30 taken from a position rotated 90° with respect to the Fig. 30 view;
Fig. 32 is an enlarged cross-sectional view of loop electrode ends of a modification;
Fig. 33 is a cross-sectional view of the electrode end of Fig. 32 taken from a position rotated 90° with respect to the Fig. 32 view;

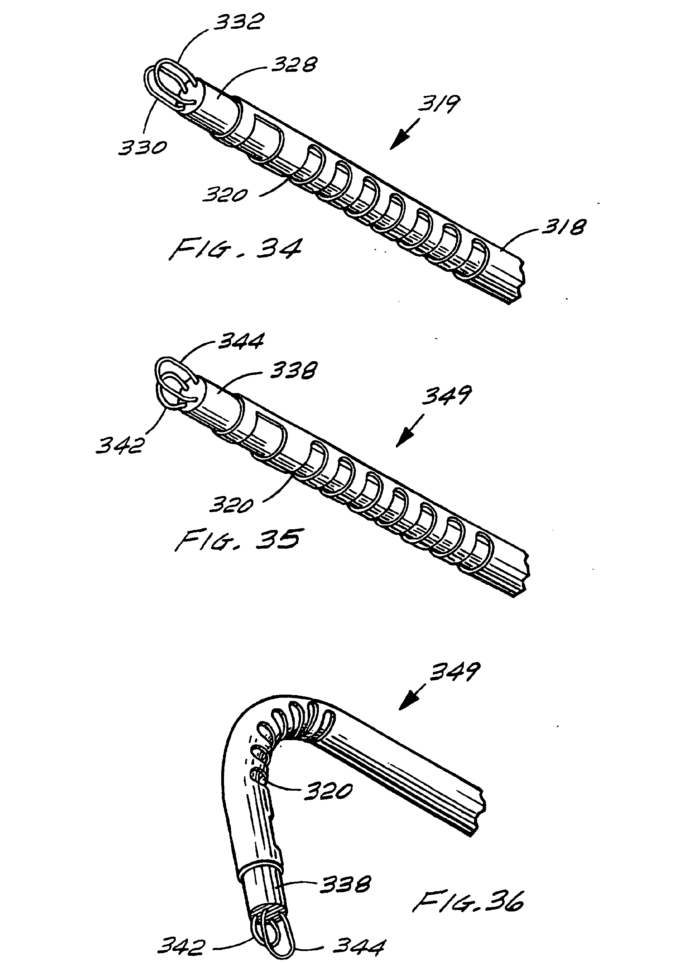

Fig. 34 is an enlarged perspective view of the working end of the handpiece of Fig. 30;
Fig. 35 is an enlarged perspective view of the working end of the handpiece of Fig. 32;
Fig. 36 is an enlarged perspective view of the working end of the handpiece of Fig. 35 but with the end in its flexed position.

### DETAILED DESCRIPTION OF THE PREFERRED EMBODIMENTS

The MIS procedures are well known in the art and need no further elaboration here except to state that the invention has to do with the construction of the electrosurgical electrode that is inserted into the cannula during the procedure for the purpose of shrinking or excising tissue or coagulating bleeders. The use of electrosurgical procedures to shrink herniated disks and other tissue is also well known in the art and also needs little elaboration here. It will suffice to state that the procedure with the novel handpiece of the present invention is similar to that using the unipolar electrode with the incorporated heat sensor, except that, with the bipolar handpiece of the invention, no complex temperature sensor and associated control circuit are needed, as, in the case of the bipolar electrode, electrosurgical currents are confined to the small active region between the electrode tips and will cause little if any heat generation at remote tissue locations.

In a preferred embodiment of the invention, an electrode designed for MIS is provided with an outer tubular housing that is stiff enough to be inserted into the cannula and has a straight end portion or tip that is capable of flexure when a bending force is applied but which end portion has inherent memory that will restore the tubular housing to its pre-flexed configuration when the bending force is removed. The tube may be made out of a plastic or a metal such as stainless steel. Examples of suitable plastics with some flexibility and inherent memory that will keep the tubing straight when the bending flex is removed are Delron, vinyls, and nylon. The location in the tubing where flexure occurs can be established in several ways, the preferred way being to weaken one side of the outer tubing, as for example by spaced slots, at an end region spaced from the tip where flexure is desired. In a preferred embodiment, a handle is provided for supporting the outer tubular housing and the handle is provided with a hand grip and a trigger that is connected to a mechanism to flex the tube end when squeezed. In a preferred embodiment, this result can be obtained by attaching a pull string or wire to the side of the tube that has been weakened by the cutting of slots but beyond the slots. The pull string or wire is attached to the trigger at the opposite end of the tube. When the trigger is squeezed against the grip, the tube end will flex in the direction of the weakened side of the tube. When the trigger is released, the tube due to its inherent memory returns back to its original straight position.

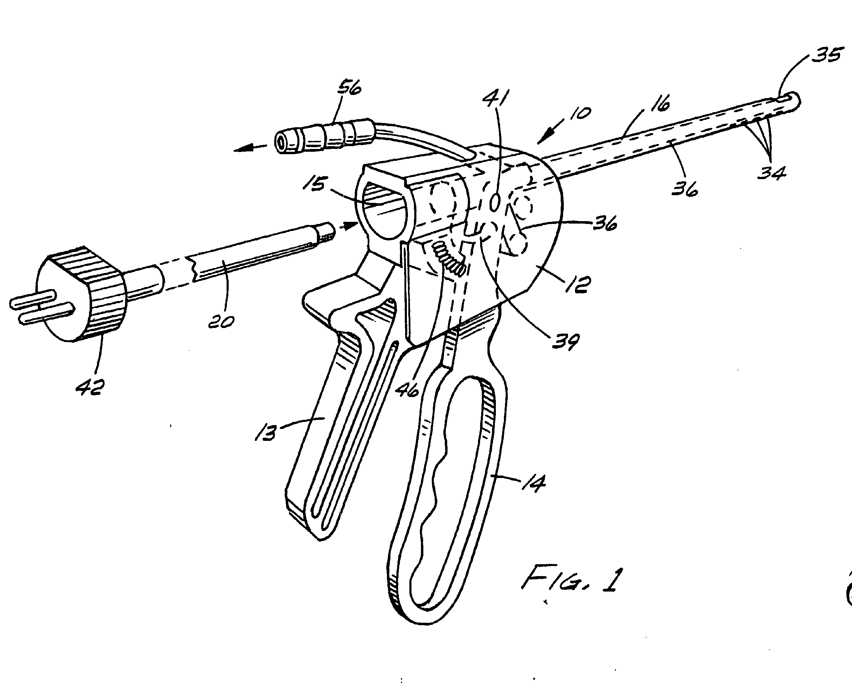

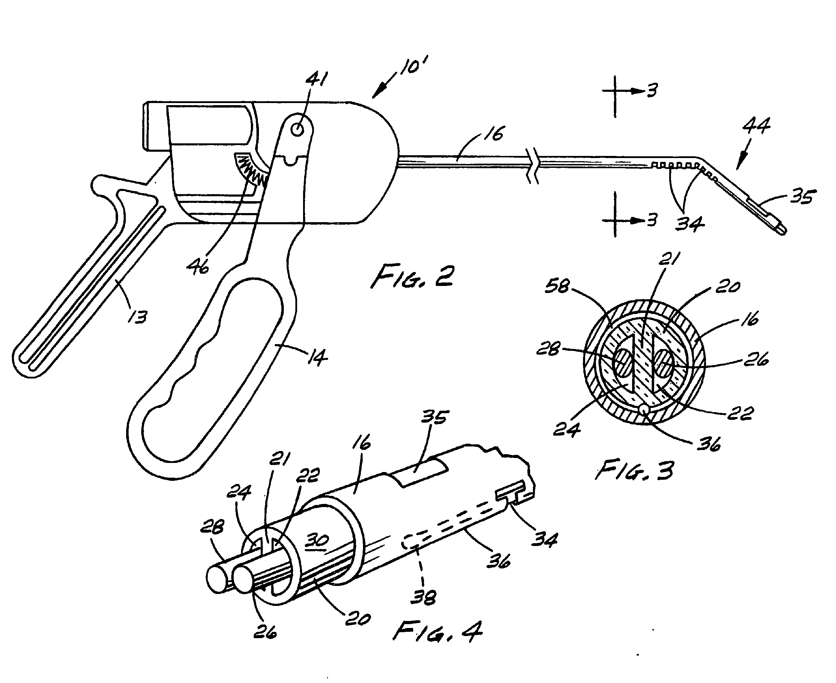

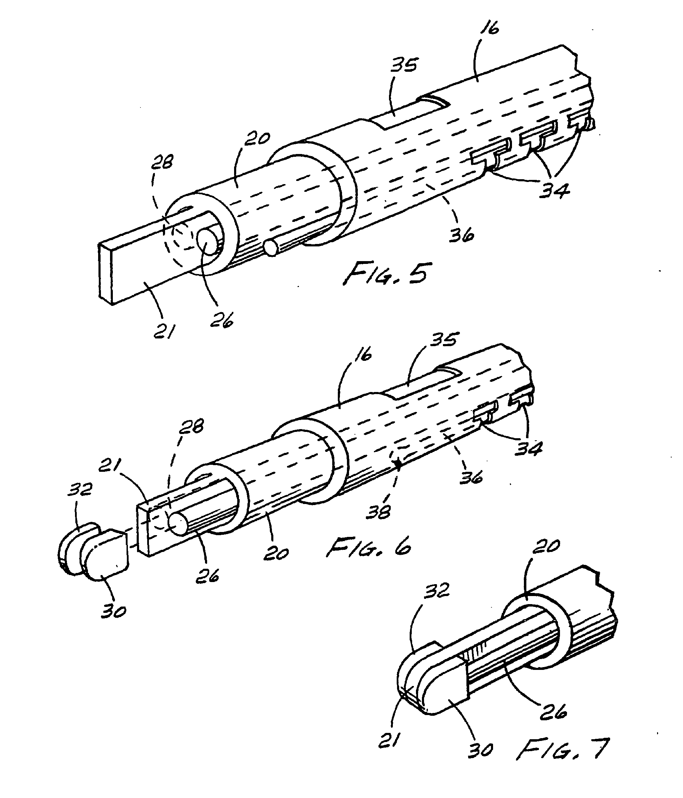

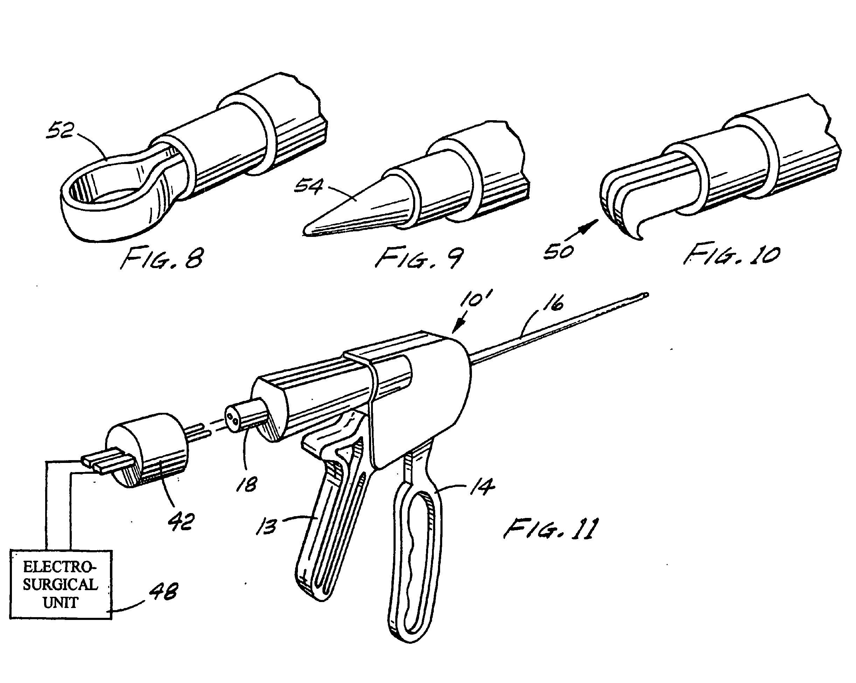

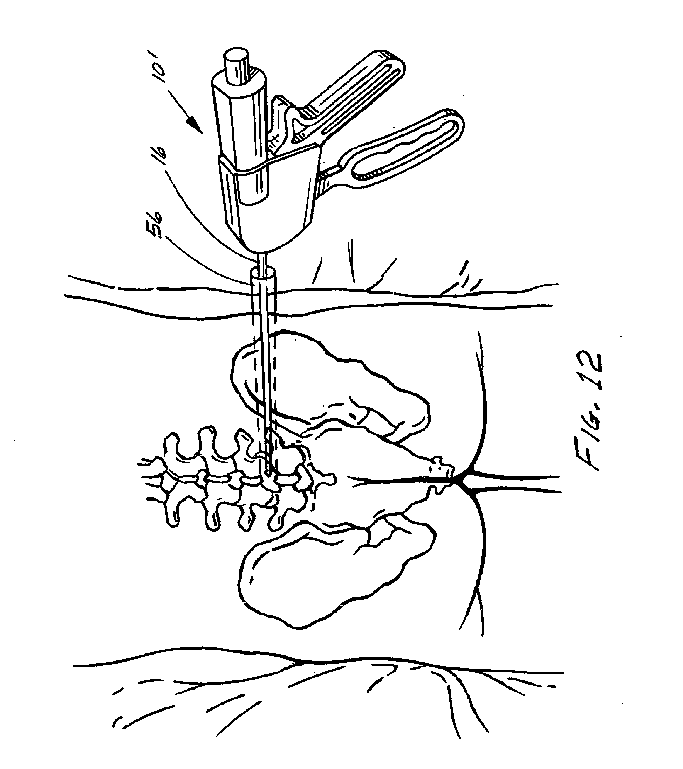

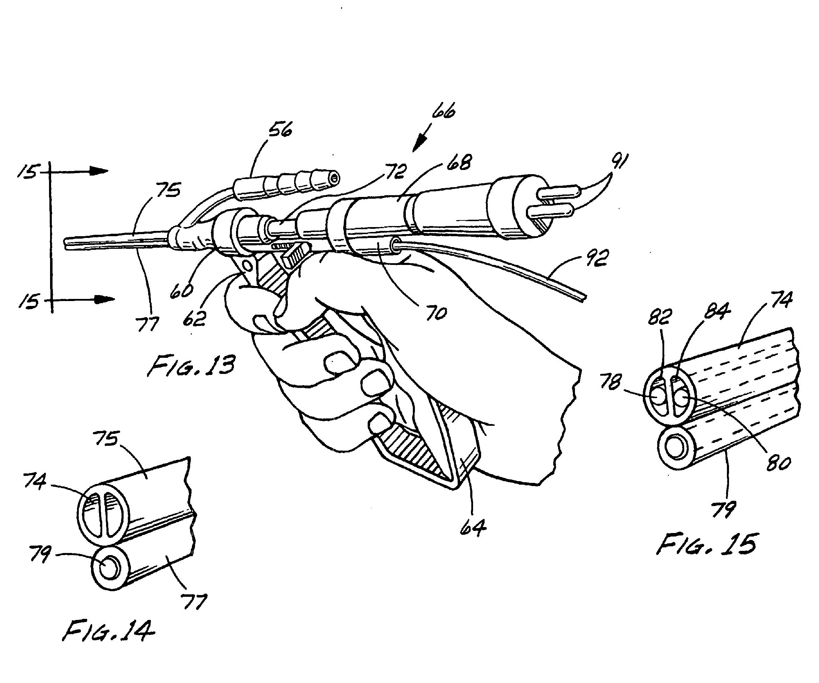

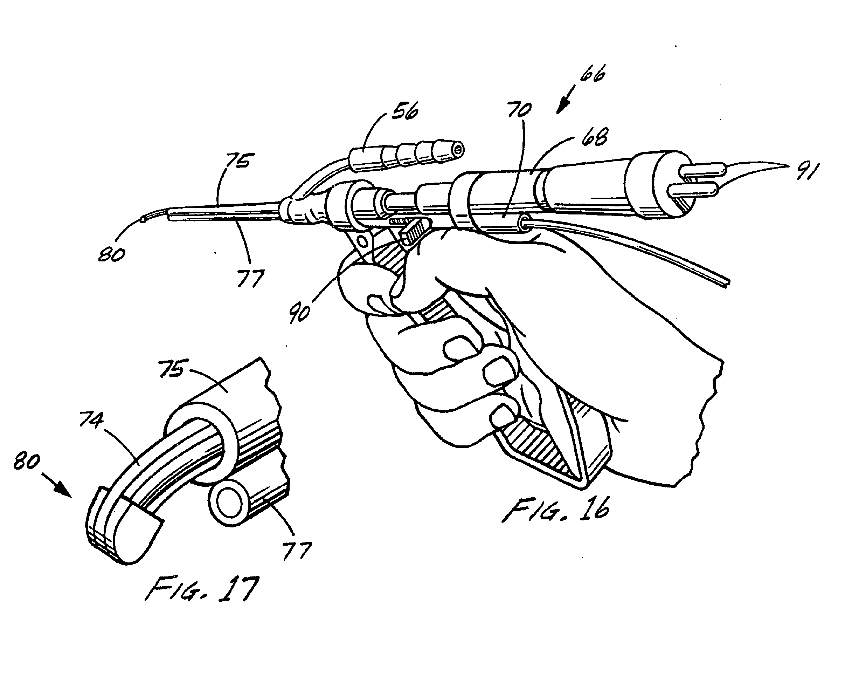

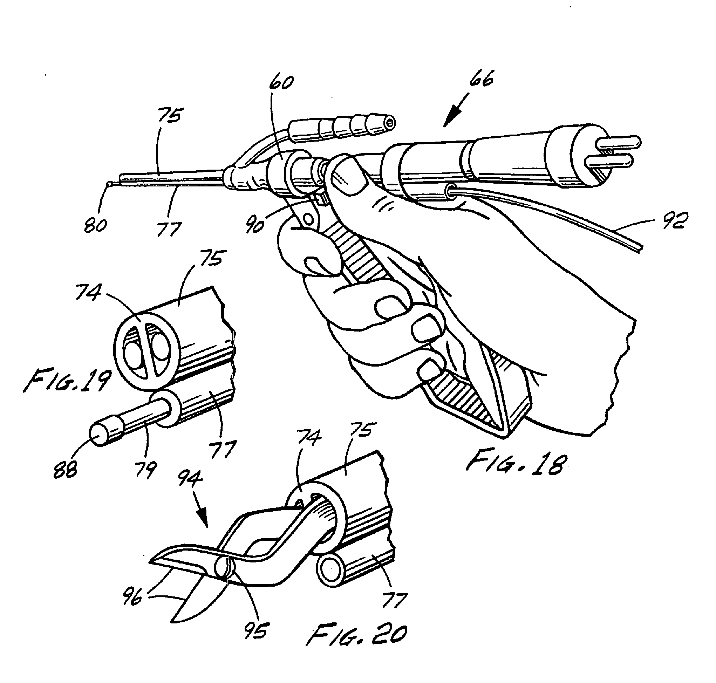

Referring now to Fig. 1, one form of bipolar handpiece according to the invention is shown at 10. It comprises a handle 12 having a grip 13, a trigger 14, and through a bore 15 at its top is mounted the outer first tubular member 16. The latter has a small outside diameter that will allow it to be inserted into the standard cannula used for MIS. A typical range is about 0.07-0.1 inches. A typical length is about 10-20 inches. The outer tubular member 16 is preferably supplied with an enlarged diameter end 18 (see Fig. 11) acting as a stop for the tubular member 16 when it is inserted into the handle bore 15 and secured therein by, for example, a set screw (not shown). This simple mounting allows the use of disposable assemblies of tubular member and electrodes if desired.

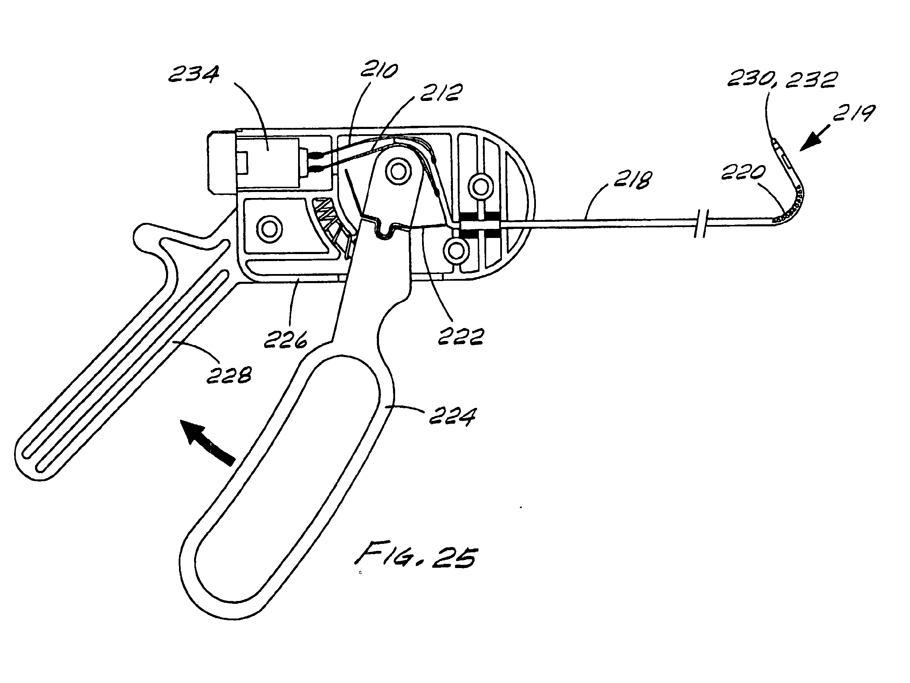

Inside the outer tubular member 16 is an inner electrically-insulating, for example, plastic, lumen (second tubular member) 20 that has an electrically-insulating wall 21 down its middle forming two electrically-insulated compartments 22, 24 (Fig. 3). The inner tubular member 20 may be secured within the outer tubular member 16 by any convenient means, such as a set screw (not shown), which, as will be explained in greater detail below, allows replacement of the second tubular member 20 with its bipolar electrode by a another bipolar electrode or a similarly-configured unipolar electrode. Fig. 1 illustrates this feature by showing an electrode 20 being plugged into the housing 12 via the opening 15 and extending into the outer tubular member 16. Two electrically-conductive wires 26, 28, for example of stainless steel, are each extended through one of the insulated compartments 22, 24, with the result that the wall 21 electrically separates the two conductive wires (Fig. 4). Additionally, the circular wall 30 surrounding the separating wall 21 for a short distance at the tube end is stripped back (Fig. 5) leaving only the separating wall 21 projecting forward. This projecting wall 21 now becomes the insulation between two half-ball electrodes 30, 32 connected as by welding to the projecting ends of the wires 26, 28 (Fig. 6).

Fig. 4 shows the remote end of the assembly before the half-ball electrodes have been added, and Fig. 6 shows the electrode wires 26, 28 which were first pulled forward to provide extra room to attach the half-ball electrodes 30, 32 to the wire ends, after which the wires are retracted pulling the half-ball electrodes back to their operative position, as shown in Fig. 7, in which the ball electrodes 30, 32 are fully spaced over their entire length (the direction of the longitudinal dimension of the assembly) by the center wall insulation 21.

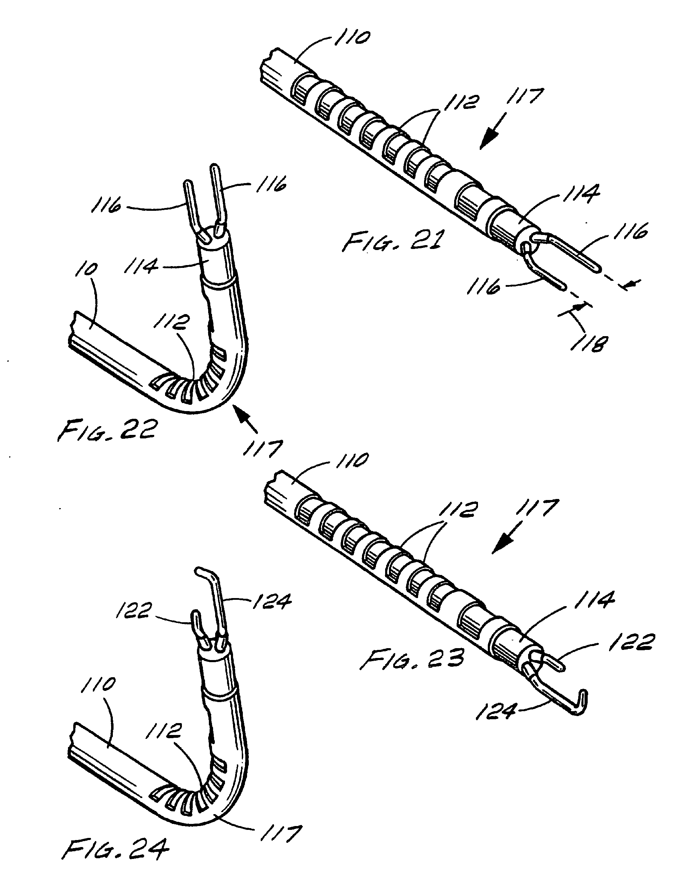

The outer tubular member 16 is weakened at a location spaced a short distance from the remote end of the tubular member 16, as by cutting a series of spaced slots 34 that extend through the outer wall 30. A pull wire 36 is extended through the outer tubular member 16 along the bottom side of and outside of the inner tubular member 20, i.e., at the same side as the slots 34, and anchored 38 (Fig. 4) to the outer wall 30 as by use of adhesive or by fusion if the outer tubular member is of plastic, or by welding if the outer tubular member is of metal. An opening 35 is made in front of the slots 34 on the top part to provide access to the pull wire end to allow this connection to be made during assembly. The opposite end of the pull wire 36 is attached 39 to the trigger 14 (Fig. 1). The outer tubular member 16 is held in a stationery position within the handle 12, and the grip 13 is likewise stationery with respect to the handle 12. Hence, when the trigger 14, which is pivotably mounted 41 on the handle 12, is squeezed, illustrated in Fig. 2, the pull wire 36 to which it is attached applies a pulling force on the remote end of the outer tubular member 16 which as shown at 44 causes it to flex downward about the weakened section 34. When the trigger is released, the natural tendency of the outer tubular member 16 to return to its normal straight position restores it to the position shown in Fig. 1. If desired, a return spring 46 can be added to the trigger 14 to aid this motion.

The inner tubular member 20 together with its insulated half-ball electrodes 30, 32 positioned at the end of this flexible tip is the actual bipolar electrode. Connected to the proximate ends of each of the wires 26, 28 is a standard bipolar connector 42 (Fig. 1). When the latter is plugged into a like connector on the front panel of a conventional electrosurgical unit, shown schematically at 48 (Fig. 11), and the unit activated, bipolar electrosurgical currents flow along the wires 26, 28 to the ball tips 30, 32 and an electrosurgical discharge is generated that extends between the ball electrodes 30, 32 around the end of the insulating wall separator 21. While it is preferred for reduction of herniated disks to use the ball electrodes, in a bipolar arrangement to confine the discharge to the immediate vicinity of the electrode ends, it will be understood that other known electrode shapes can be substituted for the ball electrodes, such as straight wires, needles, hooks, or loops. In addition, a feature of the invention is that the dual tubular member assembly makes it particularly easy to accommodate other electrode ends, by sliding out the inner tubular member 20 (see Fig. 1) from the outer tubular member 16 and sliding in its place another inner tubular member 20 with a different electrode configuration. This can be done before the outer tubular member 16 is extended through the cannula in the patient or even while the cannula is in place within the patient. In addition, a bipolar assembly in its dual lumen arrangement can be replaced by a unipolar electrode in a lumen with only a single compartment, in which case, the unipolar electrode end, with one of the aforementioned electrode shapes, would project forward from the end of the inner tubular member 20', which in this case would not require the center insulation present. This is illustrated in Fig. 9. When an electrode substitution is to be made, the surgeon can pull out the inner tubular member and replace it with another inner tubular member with a different electrode thereby enabling the surgeon to change electrodes during the procedure without removing the handle with its outer tubular member that has already been strategically placed in the surgical site. While a range of electrosurgical current frequencies can be employed, it is preferred that the frequency range employed be preferably in the range between 1.5 and 4 MHz.

Figs. 8 and 10 illustrate other active electrode configurations. Fig. 10 shows a bipolar hook assembly 50, which can also easily be made unipolar. Fig. 8 shows a unipolar loop assembly 52. Fig. 9 shows a unipolar needle 54 or pointed electrode assembly.

Among the benefits of this invention is that it offers the surgeon control and flexibility during surgeries that require difficult placement of electrodes and also movement of the active end at the active surgical area while the electrode is within the cannula to perform precision surgery. The degree to which the surgeon actually needs to bend the flexible tip depends upon its location relative to the disk area to be cut or shrunk. The flexible tip provides the surgeon with an additional degree of freedom in finding the optimum electrode position before energizing the electrosurgical unit. The long nozzle 16 is needed because, typically, the cannula 50 is inserted from the patient's side (Fig. 12), and the cannula 56 is positioned while the surgeon is observing the position of the cannula though the viewer. After the cannula is positioned properly, then the electrode 16 can itself be pushed through the cannula 56 until its flexible end 44 is outside the end of the cannula and further positioned within the disk surgical site by moving it forward or backwards and by flexing the electrode tip 44.

The connector 42 can be plugged directly into the electrosurgical mainframe 48, and thus the electrosurgical energy furnished at the electrode working end will be determined by the mainframe controls and a conventional footswitch. However, most surgeons prefer hand control of the electrosurgical energy, as was explained in the earlier referenced patent, which is easily accomplished by using the finger switch activator described in that earlier referenced patent and which can be mounted on the cannula or handle.

Fig. 1 shows a variant 10 in which the inner tubular member 20 is replaceable, and also is provided with a suction tube 56 connected to the handle and to the clearance space 58 (Fig. 3) between the inner and outer tubular members. When suction is provided at the end 56, it is delivered to the remote end of the outer tubular member 16 at the surgical site and thus will exhaust any fumes or smoke that may interfere with the surgeon's vision as well as cool the surgical site. The variant 10' of Figs. 2 and 11 include a fixed inner tubular member 20 and lacks the suction feature.

While the parts of the electrosurgical handpiece, made up of metal and if of plastic, of Delrin for example, are autoclavable, the device is sufficiently simple that it can be manufactured at very low cost with a less expensive plastic and thus can be made disposable.

The handpiece of the invention is generally useful for treating tissue and is useful in particular in the following situations. Thermally induced radiofrequency for shrinkage of collagen, shrinkage and tissue denaturazation, and collagen contraction. For treating and producing profound shrinkage of capsular tissue, for example, due to denaturazation of collagen fibers. Also to shrink and remodel collagen fibrils after exposure to high frequency radiowave energy. As a further example, shrinkage of collagen to promote capsular stability has been shown to be effective for shoulder dislocations and herniated discs, as examples.

Figs. 13-19 show a modification according to the invention comprising both a bipolar and a unipolar electrode, with the additional feature of allowing the surgeon to selectively choose and activate a bipolar or a unipolar electrode. This embodiment also includes as an additional feature electrodes with pre-configured ends such as pre-bent ends which are normally inside of a relatively stiff outer tube but which when extended outside of the outer tube will assume its pre-bent position. One form of this embodiment comprises a handle 60 to which is attached a hand grip having a forward portion 62 affixed to the handle 60 and a rearward portion 64 which is secured to a slidable stiff member 66 comprising an upper part 68 and a lower part 70. The upper part 68 is connected to a relatively stiff inner tube 72 which is slidable within the handle 60 and which is connected to and functions to push forward, when the grip is squeezed, a dual lumen 74 of the type illustrated in Fig. 7 together with its wires 78, 80 of a bipolar electrode 80 that may be of the half-ball type shown in Fig. 7 which extend through the dual compartments 82, 84 of the relatively stiff electrically-insulated inner first plastic tube 74. The latter is slidably mounted within a stiff outer tube 75. Below the latter is mounted a stiff second tube 77 which slidably houses a slidable electrically-insulated tube 79 housing a wire 81 connected at its end to an unipolar electrode 88. Fig. 15 shows just the two side-by-side electrically-insulated tubes 74 and 79 without the outer housings 75, 77.

Normally, both the bipolar and the unipolar electrodes are in their retracted position shown in Figs. 13 and 14. When the grip is squeezed, the member 72 is pushed forward toward the working end causing the dual lumen 74 to which it is connected, together with its bipolar wires to which the bipolar electrode 80 is connected, to project forwardly out of their housing 74 as shown in Figs. 16 and 17. The surgeon can then activate the electrosurgical unit to supply via terminals 91 bipolar electrosurgical currents to the bipolar electrode end 80. When the grip is released, the spring tension of the grip, or an additional spring (not shown) if needed, will cause the tube 74 to retract to the position shown in Figs. 13 and 14. When it is desired to operate the unipolar electrode, the surgeon presses a slide 90 on the side of the tube 70, which slide is connected to the single tube 79, which causes it to move forwardly from its retracted position (Fig. 14) to its extended position shown in Figs. 18 and 19. This action causes the unipolar electrode 88 to project forwardly. The surgeon can then activate the electrosurgical unit via wire 92 to supply unipolar electrosurgical currents to the unipolar electrode end 88. The two electrode movements are separate from one another so that the surgeon can selectively choose which electrode to use in the procedure. When the surgeon releases the slide 90, a spring (not shown) causes the single tube 79 to retract within its outer tube 77.

Preferably, at least the projecting end of one, preferably both, of the respective bipolar/unipolar tube is made of a material that can be pre-bent and has sufficient memory to retain its pre-bent shape when extended outwardly from its restraining outer tube. Either a plastic can be used or a metal, such as stainless steel, which has been treated, as by tempering, to retain a pre-bent shape. This is well known in the art and suitable materials will be apparent to those in this art. If a metal is used for the inner tube, the electrode wires will have to be suitably insulated from one another or alternatively, the tube can be lined with an insulating layer. In this embodiment, with electrodes having a pre-bent and thus fixed shape, it is not possible to change their orientation, i.e., whether straight or the angle of the bending. The only control that the surgeon has is over the length of the extension. A feature of the invention is a family of unipolar and bipolar electrodes with differently-oriented ends, some bent in one direction, others bent is still other directions, and some straight. Fig. 17 shows one member of the family with an end that bends to the left. Fig. 19 shows a member of the family with an end that is straight. Typically, the family would contain either bipolar or unipolar electrodes or both with ends that are pre-bent in all four directions as well as straight. As in the Fig. 1 embodiment, new electrodes 74, 79 are easily plugged into the end of their respective tubular housing handle as needed, which can be done during the procedure. With the bipolar electrode, the replacement electrode telescopes through tube 68, so that when the latter is moved relative to grip 62, the entire tube 72 moves with it. With the unipolar electrode, as one example, the side can be provided with a rack (visible through the side slot in Fig. 13), with the slide 90 fitted with a suitable gear or other means to move the replacement tube 77 with the slide. The electrodes can be made at a relatively low cost and thus can be made disposable. The more expensive handle can be reused. Other handle constructions that allow extension and retraction of replaceable electrodes will be apparent to those skilled in this art.

Another example according to the invention of a replacement element for the bipolar or unipolar housing is shown in Fig. 20. In this case, the new structure is the same as that of Fig. 13 except that if desired the unipolar structure can be omitted. Only the working end is shown in Fig. 20, which in this embodiment is a scissors 94 comprising bent stainless steel scissor elements 96 connected at the gun end to the tube 72 and at the remote end connected by a pivot 95. As will be noted, the scissor elements 96 when extended out of their tube 75 are pre-configured to assume an open position. The grip 62, 64 (Fig. 13) is spring loaded so that the surgeon can extend the scissor elements 96 as shown by squeezing the grip. When the grip is released, the spring action causes the scissor elements to be retracted, the confining action of the walls of the tube 75 forcing the scissor ends together cutting in a normal scissor action any tissue around which the scissor ends have been placed. The dual lumen 74 allows bipolar currents to be applied to the scissor ends if desired, in which case the pivot 95 would have to electrically-insulate the two scissor elements, as with washers for example. The scissors 94 can also be configured for mounting in the unipolar tube 77 in which case unipolar currents can be applied to the scissor elements 96. It is also possible to have no terminals connected to the scissors 94 so that no electrosurgical currents can be applied thereto, in which case the scissors 94 can be operated just with a mechanical action. As before, the scissors 94 can have its own tubular housing 74 and thus be plugged into the gun of Fig. 13 to replace a bipolar or unipolar electrode, all while the tubular structure remains within the cannula during the procedure. It is preferred that the scissor end is pre-configured as in the Fig. 13 embodiment so that it can be caused to assume a particular orientation when extended, with the possibility of repalcing it with a scissors of a different orientation if needed. Or if desired, it can be mounted as in the Fig. 1 embodiment with means for flexing the remote end of the structure. Other usable mechanical or electrical structures will be appreciated by those skilled in this art.

Figs. 21-24 describe two additional bipolar electrodes for use in the handpiece of the invention. The two new bipolar electrodes are configured to provide hemostasis or cauterization of a bleeder. For this purpose, the electrode ends comprise two electrically-insulated, adjacent, prong-like elements whose ends are laterally spaced apart a distance sufficient to embrace a typical blood vessel in a patient. A preferred spacing is about 2 mm. The spaced prongs project from the flexible end of its tubular housing, and can be positioned by moving the tubular housing and flexing the tubular housing tip to surround or flank the blood vessel to be cauterized by the surgeon manipulating the tubular housing and flexible tip as described above. In a first preferred embodiment, the prong ends are straight and lie in a plane extending substantially perpendicular to the plane of flexing. In a second preferred embodiment, one prong end is straight and the other forms a hook lying in a plane extending substantially parallel to the plane of flexing. These embodiments can also make use of the interchangability of electrodes as described above. So, for example, after first using a bipolar or unipolar electrode for a cutting procedure, either of the electrodes of the present invention can be substituted to perform hemostasis of any blood vessels cut during the procedure.

The gun configuration remains the same. The only changes made are the construction of the bipolar electrode end. Two electrically-insulated wires (not shown) are passed through insulated compartments (not shown) of a tubular housing 100 (Fig. 21) whose end is weakened as by spaced slots 112. A third wire (also not shown) is connected to the housing end such that when the wire is pulled by the surgeon, the housing end can be flexed as shown in Fig. 22. The two wires are not only insulated from each other so that bipolar electrosurgical currents can be applied between them, but they are also insulated from the tubular housing 10 which may be of metal. The latter insulation may be in the form of a flexible plastic tube 14. The two wires terminate in a pair of metal prongs 116 which project from the end of the plastic tube 114 in parallel relationship. The prong ends are laterally spaced from one another, indicated by 118, a distance sufficient to surround on opposite sides a blood vessel. A spacing 118 of about 2 mm is preferred. The surgeon can manipulate the position of the electrodes by pulling on the flexing wire. Fig. 22 illustrates one possible position of the prong ends 116 when the flexible tip 117 is bent. It will be noted that, in this embodiment, the plane of flexing, shown vertical in Fig. 22, is approximately perpendicular to the plane occupied by the two prongs 116, shown horizontal in Fig. 21.

In the variant shown in Figs. 23 and 24, the prong ends are oriented in a vertical plane, and when the electrode end 117 is flexed, the plane of flexing, vertical in Fig. 24, is parallel to the plane of orientation, vertical in Fig. 23. This demonstrates that the prong ends can be oriented in various planes with respect to the plane of flexing. The orientation is thus not critical. In the Fig. 23 variant, one projecting prong 122 is short, and the other projecting prong 124 is longer and the end 126 is hooked back toward the short projecting prong 122. This hook arrangement may make it easier in certain circumstances to trap and embrace a blood vessel which needs cauterization.

Once the surgeon has positioned the prong ends around the blood vessel, he or she then activates the electrosurgical apparatus causing a bipolar discharge between the bare prong ends and causing cauterization of the blood vessel in the usual way. Other usable mechanical or electrical structures following the teachings of the prior application will be appreciated by those skilled in this art. As with the previous embodiments, the insulating tube 14 will prevent accidental touching of patient tissue by the prong sides, so that the bipolar discharge is locallized to the spacing beteen the prong ends.

In the previous embodiments, the handpiece end is made flexible by providing a spaced set of slots at the handpiece end, such that, when a pulling wire attached to the handpiece end is pulled by the surgeon, the end bends or flexes in a direction toward the slotted side. Figs. 25-29 illustrate preferred embodiments in which the handpiece end constructions are configured to provide easier flexing of the handpiece end, or more controlled flexing and positioning of the handpiece end. In a first preferred embodiment, the slots have tapered flanks, such that the open sides of the slots are wider than the closed sides. In a second preferred embodiment, the slots vary in depth as they approach the handpiece end. In a third preferred embodiment, the slots are replaced by a spiral end of the handpiece. In a fourth preferred embodiment, the slots are replaced by a spring.

As in the earlier embodiments, for the bipolar handpiece, two electrically-insulated wires 210, 212 are passed through insulated compartments of a tubular housing 218 whose end 219 is weakened as by spaced slots 220 and thereby made flexible. A third wire 222 is connected to the movable trigger 224 of a gun-type housing 226 having a fixed handle 228. The two wires 210, 212 are not only insulated from each other so that bipolar electrosurgical currents can be applied between them, but they are also insulated from the tubular housing 218 which may be of metal. The two wires 210, 212 may terminate in various unipolar or bipolar electrode shapes as described above. It will be noted that, in this embodiment, the plane of flexing, shown vertical in Fig. 25, is approximately a vertical plane through the center of the gun and the center of the slots 220. The two insulated wires 210, 212 terminate at the left hand of the gun in a connector 234 having at its left end prongs (not shown) to which can be plugged the standard bipolar cable which connects the gun to electrosurgical apparatus. The pulling wire 222 is anchored as by welding (not shown) to the flexible end at, for example, a top point (when the bending is to occur upward as shown). The welding is accomplished via an access slot 236 in the end of the tube 218 beyond the bending slots 220 before the insulated tube with the bipolar electrode wires is inserted. When the surgeon while holding the gun handle 28 squeezes the trigger 24, the wire is pulled flexing the tubing end 219 as shown in Fig. 25. The position of the pulling wire and that of the slots can be oriented in various planes to control the plane of flexing.

These embodiments have to do with modifying the construction of the flexible end 219 of the tubular housing 218 which is typically of metal in order to improve its flexing ability, its lifetime, and the position control available to the surgeon during use. In the four embodiments to be described, only the flexible end of the tubular housing is shown, the internal elements being omitted because they can be the same as described above. In these four embodiments, the tubular housing can be of relatively stiff metal that will not bend except where desired at the area of the slots 219. For example, a suitable metal is stainless steel and a suitable tube wall thickness is about 0.002-0.01 inches. The tube outside diameter is typically about 0.04-0.1 inches. The first two embodiments still employ slots to furnish the flexibility, but of different configurations, while the last two embodiments substitute a generally spiral construction for the slots. For the application of shrinking herniated tissue via a cannula, the tubular housing is typically about 15-20 inches long.

In the first embodiment of Fig. 26, a one-piece tubular housing 240 is provided with the access slot 236 and with the bending slots 242 having tapered side walls 244 with the open side 246 of each slot being wider than the closed side 248. This construction allows more bending of the tip 248 before the slot walls engage, compared with a slot construction in which the slot walls are parallel.

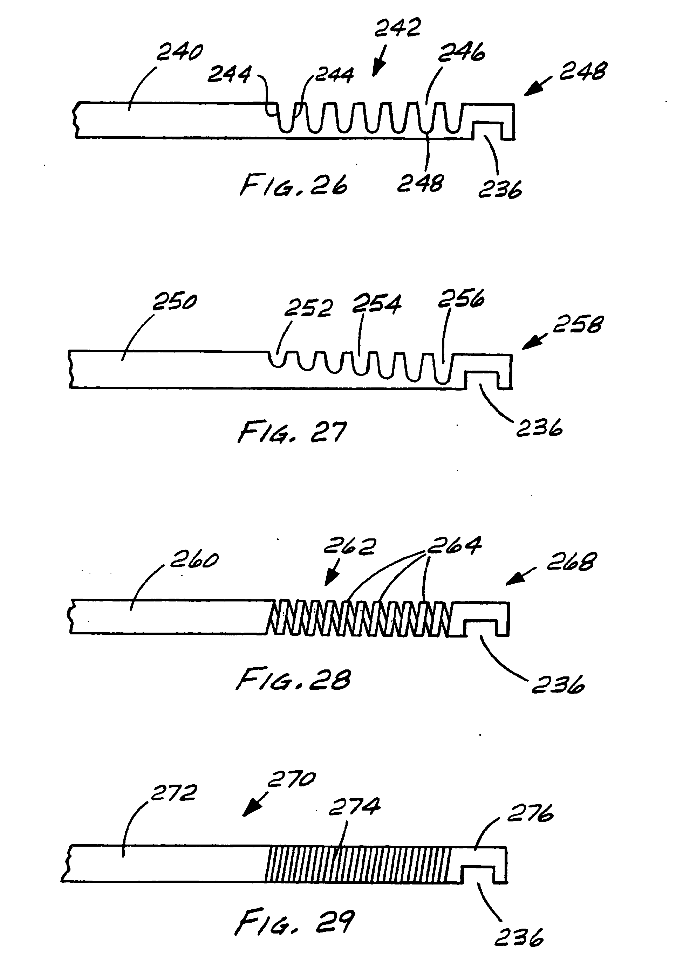

In the second embodiment of Fig. 27, a one-piece tubular housing 250 is provided with the access slot 236 and with the bending slots 252-256 varying in depth as the tip 258 is approached. This construction again allows more bending of the tip 258 before the slot walls engage since the slots 256 furthest from the handle 228 tend to bend more than the slots 252 which are closer.

It will also be understood that the tapered-wall slots, the parallel-wall slots and the varying-depth slots can be mixed and matched depending upon flexing needs of the handpiece tip. So, for example, the flexible end can comprise one or two parallel-wall slots followed by six-eight tapered-wall slots closer to the tip, with or without addition of the varying slot depths.

In the third embodiment of Fig. 28, still a one-piece tubular housing 260 is provided but, instead of slots being cut into the top wall of the tube, a spiral 262 with about, for example, eleven turns 264 is cut into the tubular member wall. The length of the spiral 262 can be about 0.5-2 inches. This construction provides a greater restoring force for restoring the tip 268 to its normal straight position after being flexed.

In the fourth embodiment of Fig. 29, the one-piece tubular housing is replaced by a tubular housing 270 comprising a steel tube 272, a spring section 274, followed by a short section 276 of the steel tube. The construction can consist of welding one end of the spring 274 to the first steel tube 272, and then welding the other end of the spring 274 to the following steel tube 276 at the tip. The advantage of this construction is that more resilience of the flexible tip can be provided by selecting a more resilient material for the flexible section than would normally be available with standard stainless steels. Also, the spiral section 274 can be given more turns and thus made more stronger than that illustrated in Fig. 28.

When a bipolar assembly is involved, then the electrode wires have to be insulated from each other and from the metal tube. The 2-compartment electrically-insulating liner tube serves this function. However, in principle, if the electrical connecting wires have their own good electrically-insulating coating, the latter serves to provide the electrical insulation and then a separate insulating liner can be dispensed with. Similarly, for the unipolar electrode assembly, if the electrical wire has a sufficiently good electrically-insulating coating, then an insulating liner to isolate same from the metal housing can be dispensed with. When a stiff electrically-insulating material, for example, of plastic, is used for the elongated tubular housing, for example, 218 in Fig. 25 or 240 in Fig. 26, then the slot embodiments of Figs. 26 and 27 can still be used and an additional insulating liner becomes unnecessary. But in this case, a plastic material will be necesary that can withstand repeated bending without breaking at the flexible tip. Such materials are well known to those in the art. The spiral embodiments of Figs. 28 and 29 can also be used with an electrically-insulating tubular housing, for example of plastic, provided that the spiral section 264 of Fig. 28 can be provided in the plastic without it breaking. The Fig. 29 embodiment can be realized by cementing a metal spring 274 to the sections 274 and 276 of plastic. Sufficiently strong commercial adhesives are available for this purpose.

In the next series of embodiments, new handpiece end constructions use the bipolar principle and are configured to provide more controlled distribution of the electrosurgical currents to the tissue to be modulated.

In a preferred embodiment, the electrode ends are formed by axially-projecting, dual spaced wire loops each connected to a terminal of the bipolar source. In a first preferred embodiment, the wire loops project in spaced parallel planes approximately the same distance from the insulated end of the electrode. In a second preferred embodiment, the wire loops project different distances from the electrode end. It is also possible within the scope of the invention for the wire loops to lie in spaced non-parallel planes.

In these embodiments, the gun configuration remains essentially the same for the bipolar version. The bipolar electrode ends are formed by spaced loops 330, 332. The two wire ends forming each loop can be spaced apart in its plane about 0.02-0.05 inches, preferably about 0.0375 inches. The radius of each loop is about one-half the latter dimension. The two loops can be spaced apart in a direction perpendicular to their respective planes about 0.013-0.025 inches, preferably about 0.0187 inches. The insulation between the two wires 330, 332 can be provided, for example, by internal glue 334, a plastic tube 336, and a heat-shrunk tube 338. Other electrically-insulating materials can be substituted. For the application of shrinking herniated tissue via a cannula, the tubular housing is typically about 15-20 inches long.

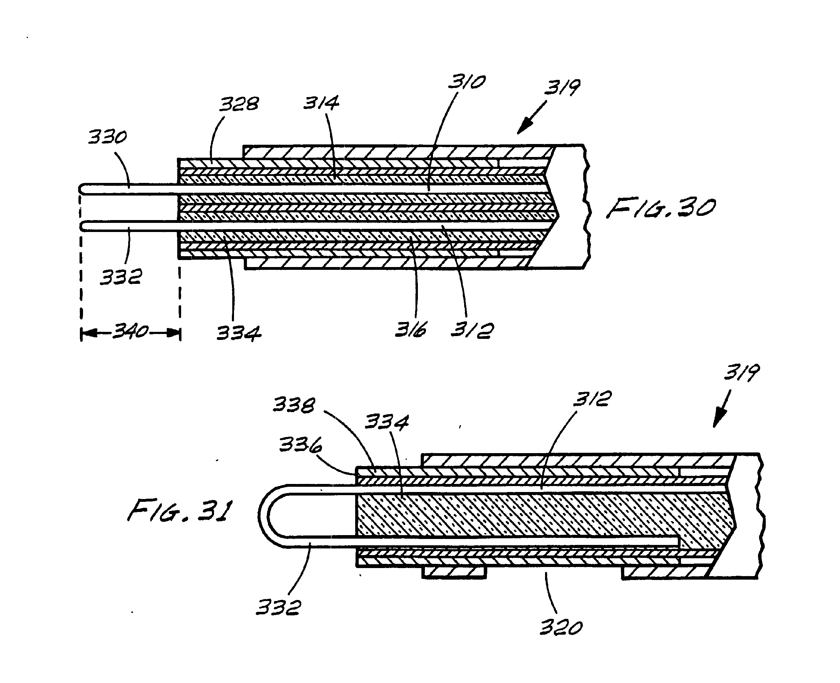

In the first embodiment of Figs. 30 and 31, the two wires 330, 332 extend the same distance from the free end of the insulating tubes 336, 338. This distance, indicated in Fig. 30 by reference numeral 340, measured along the longitudinal axis of the tube 328, is about 0.05-0.09 inches, preferably about 0.075 inches.

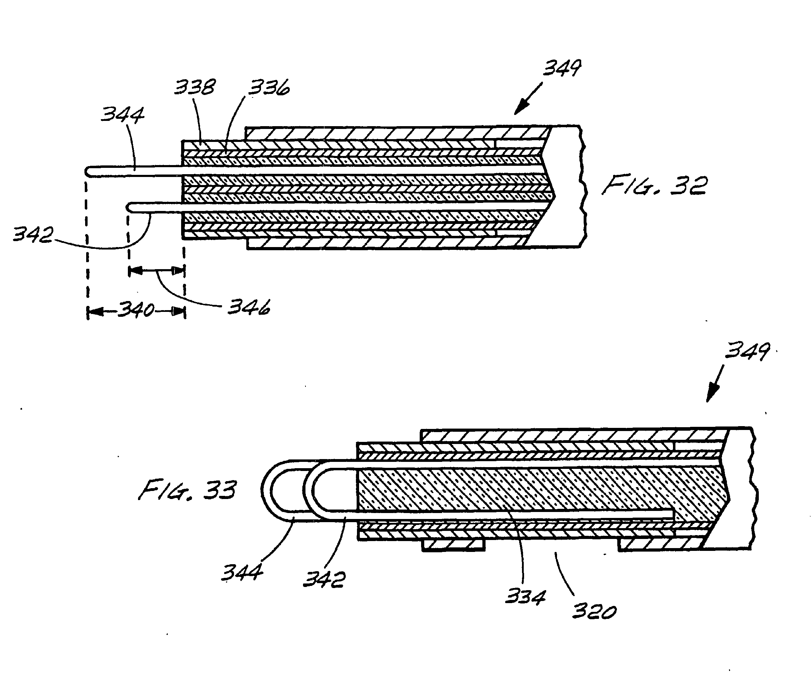

In the second embodiment of Figs. 32, 33, 35 and 36, the two wires 342, 344 are offset axially from one another in the two parallel planes and thus extend different distances from the free end of the insulating tubes 336, 338. One wire 344 extends, for example, about the same distance 340 as the wires in the first embodiment, namely, about 0.05-0.09 inches, preferably about 0.075 inches. The other wire 342 extends from the free end of the insulating tubes 336, 338 about one-half the distance, i.e., a distance 346 of about 0.02-0.045 inches, preferably about 0.0375 inches. Fig. 35 shows the second embodiment in its non-flexed position. Fig. 36 illustrates one possible position of the bipolar electrodes 342, 344 when the flexible tip 349 is bent. It will be noted that, in this embodiment, the plane of flexing, shown vertical in Fig. 36, is approximately a vertical plane through the center of the gun and the center of the slots 320.

The slots 320 can be, as described above, tapered-wall, parallel-wall or varying-depth slots, or of a spiral or a spring section. The fine loop wire used can be round with a diameter of of about 0.007-0.035 inches, preferably about 0.015 inches.

During operation of the handpiece according to these embodiments, the electrosurgical currents are concentrated between the sides of the bare wire ends 330, 332 across which the active voltage is developed. When the wires 342, 344 are offset, as in the second embodiment, an oblique discharge occurs mostly between the curved distal ends. The electrode is chosen by the surgeon for obtaining the best results during the excision of tissue or blood vessel coagulation.

While the invention has been described in connection with preferred embodiments, it will be understood that modifications thereof within the principles outlined above will be evident to those skilled in the art and thus the invention is not limited to the preferred embodiments but is intended to encompass such modifications.

## Claims

1. An electrosurgical bipolar handpiece (10) comprising:
(a) an elongated tubular first member (16) having a first end and a remote flexible second end;
(b) an elongated tubular second member (20) located within the first member (16), said second member (20) comprising an electrically-insulating tube having a first end and a remote flexible second end adjacent the first member's second end;
(c) first means (42) connected to the first member (16) at its first end for applying electrosurgical bipolar currents to first and second electrically-conductive wires (26, 28);
(d) second means (14, 36) connected to the first member (16) at its first end for selectively flexing the remote flexible end of the first member (16) relative to the first end of the first member (16) together with the remote flexible end of the second member (20);
(e) active electrically-insulated electrosurgical electrodes (30, 32) connected to the fi rst and second wires (26, 28) and extendable at the remote flexible end, said electrosurgical electrodes (30, 32) flexing together with the remote flexible ends when the second means (14, 36) is operated while maintaining them electrically-insulated; **characterised by**
(f) electrically-insulating dividing means (21) dividing the second member (20) into first and second side-by-side compartments (22, 24), wherein the first and second wires (26, 28) are each positioned, respectively, in the first and second compartments and are electrically-insulated from each other.

2. An electrosurgical bipolar handpiece (10) as claimed in claim 1, wherein the dividing means (21) comprises an electrically-insulating wall portion (21) which projects outwardly from the remote end of the second member, the electrodes (30, 32) being positioned on opposite sides of the projecting wall portion (21).

3. An electrosurgical bipolar handpiece (10) as claimed in claim 1, wherein the second means (14, 36) comprises a pull wire or string (36) positioned inside of the first member (16) and connected at one end to the remote end of the first member (16), further comprising means (14) for pulling the other end of the pull wire or string.

4. An electrosurgical bipolar handpiece (10) as claimed in claim 1, wherein said electrosurgical electrodes (30, 32) comprise first and second bare prongs laterally spaced apart by a distance approximating the size of a blood vessel in a living patient.

5. An electrosurgical handpiece (10) for MIS use as claimed in claim 4, wherein the prongs comprise a first prong and a second prong with the second prong extending further away from the tubular housing than the first prong.

6. An electrosurgical bipolar handpiece (10) as claimed in claim 1, wherein each of the first and second wires (26, 28) have a bare end forming a loop projecting out of the first member (16) at its second end, the bare end loops of the first and second wires extending in spaced planes that are parallel or non-parallel, wherein electrosurgical currents are generated between the spaced bare end loops when an electrosurgical voltage is applied to the first and second wires (26, 28).

7. An electrosurgical bipolar handpiece (10) as claimed in claim 6, wherein the loops of each of the first and second wires (26, 28) project axially about the same distance from the end of the first member (16) or different distances from the end of the first member (16).

8. An electrosurgical bipolar handpiece as claimed in claim 1, wherein the electrodes (30, 32) each comprise a half-ball, each of the first and second wires (26, 28) being connected respectively to one of the half-ball electrodes (30, 32).

9. An electrosurgical bipolar handpiece as claimed in claim 1, wherein the electrodes each comprise one of two pivotably connected scissor blades, each of the first and second wires being connected respectively to one of the scissor blades.

10. An electrosurgical bipolar handpiece as claimed in claim 9, further comprising an additional tubular member adjacent the one of the tubular first and second members and housing a unipolar electrode, and third means for selectively making the bipolar and unipolar electrodes accessible at the remote second end and for selectively energising the selected electrodes by applying thereto electrosurgical currents.

11. An electrosurgical bipolar handpiece as claimed in claim 1, further comprising an additional tubular member adjacent the one of the tubular first and second members and housing a unipolar electrode, third and fourth means for selectively advancing and retracting the respective bipolar and unipolar electrodes causing the respective ends thereof to project outwardly.

## Patentansprüche

1. Elektrochirurgisches bipolares Handstück (10) umfassend:
(a) ein verlängertes, röhrenförmiges erstes Glied (16) mit einem ersten Ende und einem fernen, flexiblen, zweiten Ende;
(b) ein verlängertes, röhrenförmiges zweites Glied (20), das innerhalb des ersten Gliedes (16) angeordnet ist, wobei das zweite Glied (20) ein elektrisch isolierendes Rohr mit einem ersten Ende und einem fernen, flexiblen, zweiten Ende aufweist, das benachbart zu dem zweiten Ende des ersten Gliedes liegt;
(c) ein erstes Mittel (42), das mit dem ersten Glied (16) an seinem ersten Ende verbunden ist, um elektrochirurgische bipolare Ströme auf einen ersten und einen zweiten elektrisch leitfähigen Draht (26, 28) anzuwenden;
(d) ein zweites Mittel (14, 36), das mit dem ersten Glied (16) an seinem ersten Ende verbunden ist, um das ferne flexible Ende des ersten Gliedes (16) bezüglich des ersten Endes des ersten Gliedes (16) zusammen mit dem fernen flexiblen Ende des zweiten Gliedes (20) selektiv zu biegen;
(e) aktive, elektrisch isolierte, elektrochirurgische Elektroden (30, 32), die mit dem ersten und dem zweiten Draht (26, 28) verbunden und am fernen flexiblen Ende verlängerbar sind, wobei sich die elektrochirurgischen Elektroden (30, 32) zusammen mit den fernen flexiblen Enden biegen, wenn das zweite Mittel (14, 36) betätigt wird, wobei sie gleichzeitig elektrisch isoliert bleiben,;
**dadurch gekennzeichnet, dass**
(f) ein elektrisch isolierendes Teilungsmittel (21), das das zweite Glied (20) in erste und zweite nebeneinander liegende Fächer (22, 24) aufteilt, wobei der erste und der zweite Draht (26, 28) jeweils in dem ersten oder dem zweiten Fach angeordnet und voneinander elektrisch isoliert sind.

2. Elektrochirurgisches bipolares Handstück (10) nach Anspruch 1, wobei das Teilungsmittel (21) einen elektrisch isolierenden Wandabschnitt (21) umfasst, der von dem fernen Ende des zweiten Glieds nach außen hervorragt, wobei die Elektroden (30, 32) auf gegenüberliegenden Seiten des hervorragenden Wandabschnitts (21) angeordnet sind.

3. Elektrochirurgisches bipolares Handstück (10) nach Anspruch 1, wobei das zweite Mittel (14, 36) einen Ziehdraht oder Strang (36) umfasst, der innerhalb des ersten Gliedes (16) angeordnet und an einem Ende mit dem fernen Ende des ersten Gliedes (16) verbunden ist, ferner umfassend ein Mittel (14) zum Ziehen des anderen Endes des Ziehdrahtes oder Strangs.

4. Elektrochirurgisches bipolares Handstück (10) nach Anspruch 1, wobei die elektrochirurgischen Elektroden (30, 32) eine erste und eine zweite blanke Zacke umfassen, die seitlich voneinander durch einen Abstand entfernt sind, der ungefähr der Größe eines Blutgefäßes in einem lebenden Patienten entspricht.

5. Elektrochirurgisches Handstück (10) für die Verwendung in der Minimalinversiven Chirurgie nach Anspruch 4, wobei die Zacken eine erste Zacke und eine zweite Zacke umfassen, wobei die zweite Zacke weiter von dem röhrenförmigen Gehäuse weg verläuft als die erste Zacke.

6. Elektrochirurgisches Handstück (10) nach Anspruch 1, wobei der erste und der zweite Draht (26, 28) jeweils ein blankes Ende aufweisen, das eine Schleife bildet, die aus dem ersten Glied (16) an seinem zweiten Ende hervorragt, wobei die blanken Endschleifen des ersten und des zweiten Drahtes in voneinander beabstandeten Ebenen verlaufen, die parallel oder nicht parallel sind, wobei elektrochirurgische Ströme zwischen den beabstandeten blanken Endschleifen erzeugt werden, wenn eine elektrochirurgische Spannung auf den ersten und den zweiten Draht (26, 28) angewendet wird.

7. Elektrochirurgisches Handstück (10) nach Anspruch 6, wobei die Schleifen des ersten und des zweiten Drahtes (26, 28) axial um etwa den gleichen Abstand von dem Ende des ersten Gliedes (16) oder um unterschiedliche Abstände von dem Ende des ersten Glieds (16) hervorragen.

8. Elektrochirurgisches bipolares Handstück nach Anspruch 1, wobei die Elektroden (30, 32) jeweils eine Halbkugel umfassen, wobei der erste und der zweite Draht (26, 28) jeweils mit einer der Halbkugelelektroden (30, 32) verbunden ist.

9. Elektrochirurgisches bipolares Handstück nach Anspruch 1, wobei die Elektroden jeweils eines von zwei drehbar verbundenen Scherenblättern umfassen, wobei der erste und der zweite Draht mit jeweils einem der Scherenblätter verbunden sind.

10. Elektrochirurgisches bipolares Handstück nach Anspruch 9, ferner umfassend ein zusätzliches röhrenförmiges Glied, das zu einem der röhrenförmigen ersten und zweiten Glieder benachbart liegt und eine unipolare Elektrode beherbergt, und ein drittes Mittel, um die bipolaren und unipolaren Elektroden an dem fernen zweiten Ende selektiv zugänglich zu machen und die ausgewählten Elektroden durch Anwenden von elektrochirurgischen Strömen darauf selektiv mit Energie zu beliefern.

11. Elektrochirurgisches bipolares Handstück nach Anspruch 1, ferner umfassend ein zusätzliches röhrenförmiges Glied, das zu einem der röhrenförmigen ersten und zweiten Glieder benachbart liegt und eine unipolare Elektrode beherbergt, und ein drittes und viertes Mittel zum selektiven Vorschieben und Zurückziehen der jeweiligen bipolaren und unipolaren Elektroden, was bewirkt, das die jeweiligen Elektrodenenden nach außen hervorragen.

## Revendications

1. Pièce à main bipolaire électrochirurgicale (10) comprenant :
(a) un premier élément tubulaire allongé (16) présentant une première extrémité et une deuxième extrémité flexible distante;
(b) un deuxième élément tubulaire allongé (20) situé à l'intérieur du premier élément (16), ledit deuxième élément (20) comprenant un tube isolant du point de vue électrique présentant une première extrémité et une deuxième extrémité flexible distante adjacente à la deuxième extrémité du premier élément ;
(c) un premier moyen (42) raccordé au premier élément (16) au niveau de sa première extrémité pour appliquer des courants bipolaires électrochirurgicaux à un premier et à un deuxième fils conducteurs de l'électricité (26, 28);
(d) un deuxième moyen (14, 36) raccordé au premier élément (16) au niveau de sa première extrémité pour exercer une flexion sélective de l'extrémité flexible distante du premier élément (16) par rapport à la première extrémité du premier élément (16) en association avec l'extrémité flexible distante du deuxième élément (20) ;
(e) des électrodes électrochirurgicales actives isolées du point de vue électrique (30, 32) raccordées aux premier et au deuxième fils (26, 28) et pouvant s'étendre au niveau de l'extrémité flexible distante, lesdites électrodes électrochirurgicales (30, 32) exerçant une flexion en association avec les extrémités flexibles distantes quand le deuxième moyen (14, 36) est mis en fonctionnement tout en les maintenant isolées du point de vue électrique ;
**caractérisée en ce que**
(f) un moyen de division isolant du point de vue électrique (21) divisant le deuxième élément (20) en un premier et un deuxième compartiments côte-à-côte (22, 24), dans laquelle le premier et le deuxième fils (26, 28) sont chacun positionnés, respectivement, dans les premier et deuxième compartiments et sont isolés du point de vue électrique l'un de l'autre.

2. Pièce à main bipolaire électrochirurgicale (10) telle que revendiquée à la revendication 1, dans laquelle le moyen de division (21) comprend une partie de paroi isolante du point de vue électrique (21) qui fait saillie vers l'extérieur depuis l'extrémité distante du deuxième élément, les électrodes (30, 32) étant positionnées sur des côtés opposés de la partie de paroi (21) faisant saillie.

3. Pièce à main bipolaire électrochirurgicale (10) telle que revendiquée à la revendication 1, dans laquelle le deuxième élément (14, 36) comprend un fil ou une corde de traction (36) positionné(e) à l'intérieur du premier élément (16) et raccordé(e) au niveau d'une extrémité à l'extrémité distante du premier élément (16), comprenant en outre un moyen (14) pour tracter l'autre extrémité du fil ou de la corde de traction.

4. Pièce à main bipolaire électrochirurgicale (10) telle que revendiquée à la revendication 1, dans laquelle lesdites électrodes électrochirurgicales (30, 32) comprennent une première et une deuxième griffes dénudées espacées latéralement l'une de l'autre d'une distance équivalente environ à la taille d'un vaisseau sanguin dans un patient vivant.

5. Pièce à main électrochirurgicale (10) pour une utilisation en chirurgie mini-invasive telle que revendiquée à la revendication 4, dans laquelle les griffes comprennent une première griffe et une deuxième griffe avec la deuxième griffe s'étendant encore plus loin de l'enveloppe tubulaire que la première griffe.

6. Pièce à main bipolaire électrochirurgicale (10) telle que revendiquée à la revendication 1, dans laquelle chacun des premier et deuxième fils (26, 28) présente une extrémité dénudée formant une boucle faisant saillie en dehors du premier élément (16) au niveau de sa deuxième extrémité, l'extrémité dénudée formant une boucle du premier et du deuxième fils s'étendant dans des plans espacés qui sont parallèles ou non parallèles, dans laquelle des courants électrochirurgicaux sont générés entre les boucles de l'extrémité dénudée espacées quand une tension électrochirurgicale est appliquée aux premier et deuxième fils (26, 28).

7. Pièce à main bipolaire électrochirurgicale (10) telle que revendiquée à la revendication 6, dans laquelle les boucles de chacun des premier et deuxième fils (26, 28) font saillie axialement à environ la même distance de l'extrémité du premier élément (16) ou à des distances différentes de l'extrémité du premier élément (16).

8. Pièce à main bipolaire électrochirurgicale (10) telle que revendiquée à la revendication 1, dans laquelle les électrodes (30, 32) comprennent chacune un demi-boulet, chacun des premier et deuxième fils (26, 28) étant raccordé respectivement à l'une des électrodes à demi-boulet (30, 32).

9. Pièce à main bipolaire électrochirurgicale (10) telle que revendiquée à la revendication 1, dans laquelle les électrodes comprennent chacune une ou deux lames de ciseaux raccordées de manière à pouvoir être pivotées, chacun des premier et deuxième fils étant raccordé respectivement à une des lames de ciseaux.

10. Pièce à main bipolaire électrochirurgicale telle que revendiquée à la revendication 9, comprenant en outre un élément tubulaire supplémentaire adjacent à celui des premier et deuxième éléments tubulaires et renfermant une électrode unipolaire, un troisième moyen pour rendre les électrodes bipolaires et unipolaires sélectivement accessibles au niveau de la deuxième extrémité distante et pour alimenter sélectivement les électrodes sélectionnées en y appliquant des courants électrochirurgicaux.

11. Pièce à main bipolaire électrochirurgicale telle que revendiquée à la revendication 1, comprenant en outre un élément tubulaire supplémentaire adjacent à celui des premier et deuxième éléments tubulaires et renfermant une électrode unipolaire, un troisième et un quatrième moyens pour avancer et rétracter sélectivement les électrodes bipolaires et unipolaires respectives provoquant les extrémités respectives de celles-ci à faire saillie vers l'extérieur.
